# NEUE EUROPÄISCHE PATENTSCHRIFT

(11) **EP 0 234 176 B2**
(45) Veröffentlichungstag und Bekanntmachung des Hinweises auf die Entscheidung über den Einspruch: **22.05.1996**
(45) Hinweis auf die Patenterteilung: 15.05.1991
(21) Anmeldenummer: 86810548.7
(22) Anmeldetag: 28.11.1986
(51) Int. Cl.: C07D 231/06, D06L 3/12, C07C 317/14

(54) **Pyrazolinverbindungen**
Pyrazoline compounds
Composés de pyrazoline

(30) Priorität: 04.12.1985 CH 5163/85; 30.04.1986 CH 1770/86
(43) Veröffentlichungstag der Anmeldung: 02.09.1987
(73) Patentinhaber: CIBA-GEIGY AG, CH-4002 Basel (CH)
(72) Erfinder: Meyer, Hans Rudolf, Dr., CH-4102 Binningen (CH)

(56) Entgegenhaltungen:
- EP-A- 0 073 996
- CH-B- 470 520
- DE-A- 2 700 996
- DE-A- 2 921 641
- DE-B- 1 469 222
- FR-A- 1 431 233

## Beschreibung

Gegenstand der Erfindung sind neue Pyrazolinverbindungen der Formel 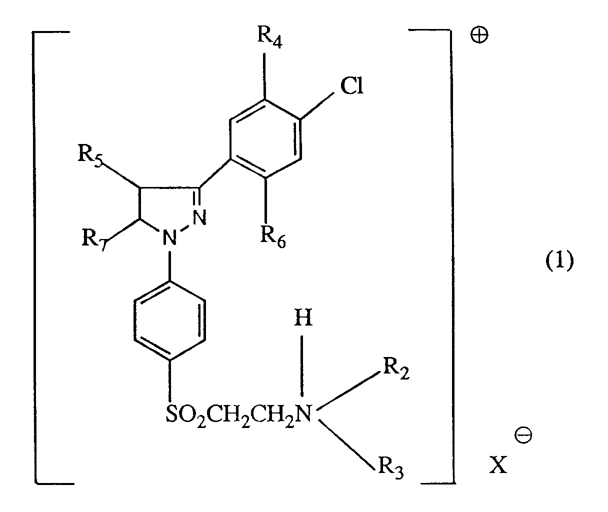
worin
R₂ und R₃ unabhängig voneinander Methyl, Ethyl oder Hydroxyethyl,
R₄ Wasserstoff oder Chlor,
R₅ und R₆ unabhängig voneinander Wasserstoff oder Methyl,
R₇ Wasserstoff, Methyl oder Phenyl, und
X^{⊖} ein Methanphosphonat-, Methansulfonat-, Phosphit- oder Sulfaminat-Anion bedeutet.

Die neuen Verbindungen der Formel (1) sind als optische Aufheller für z.B. Textilfasermaterialien geeignet.

Den neuen Salzen zugrundeliegende Pyrazolinbasen sind aus CH-A 455 802 und GB-A 1 204 953 bekannt.

Aus der CH-A-470 520 ist ferner bekannt, Δ₂-Pyrazoline als optische Aufheller für Textilmaterialien zu verwenden. Ihre Applikation erfolgt z.B. in Form von hochverdünnten organischen oder anorganischen Säuren, beispielsweise Ameisensäure, Essigsäure oder Salzsäure.

Die Erfindung bezieht sich vorzugsweise auf Verbindungen der Formel 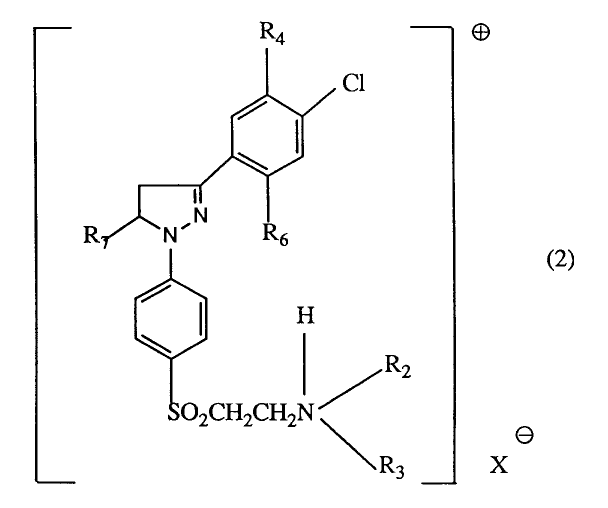
worin R₂, R₃, R₄, R₇ und X^{⊖} die zur Formel (1) angegebene Bedeutung haben.

Besonders bevorzugte Pyrazolinverbindungen sind solche der Formel 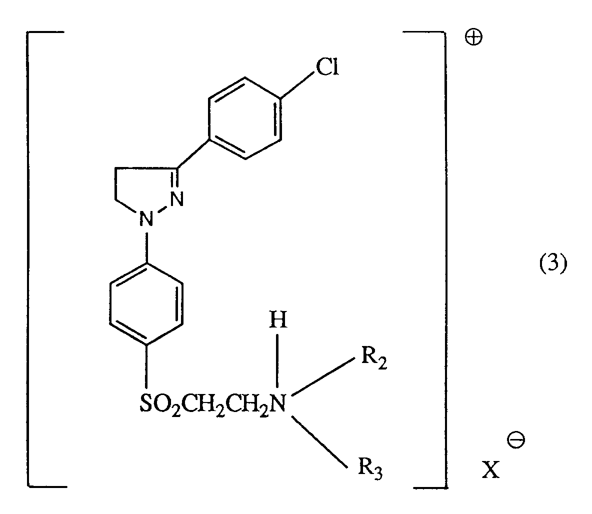
worin R₂, R₃ und X^{⊖} die zur Formel (1) angegebene Bedeutung haben.

Besonders vorteilhafte Eigenschaften weisen Pyrazolinsalze der Formel (3) auf, worin R₂ und R₃ beide Methyl und X^{⊖} ein Methanphosphonat-, Methansulfonat-, Phosphit- oder Sulfaminat-Anion bedeuten, wobei solche mit einem Phosphit- oder Sulfaminat-Anion bevorzugt sind.

Die Pyrazolinverbindungen der Formel (1) können dadurch hergestellt werden, dass man Pyrazoline der Formel 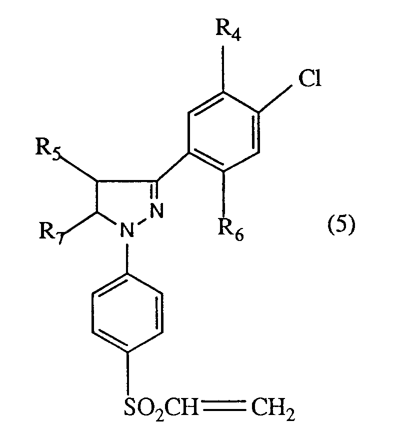
mit Dialkylaminen der Formel HNR₂R₃ umsetzt und anschliessend mit Säuren der Formel HX protoniert, wobei R₂ bis R₇ und X die zur Formel (1) genannte Bedeutung haben.

Gemäss einem bevorzugten weiteren Verfahren lassen sich die Pyrazolinverbindungen der Formel (1) durch Umsetzung von Ketonen der Formel 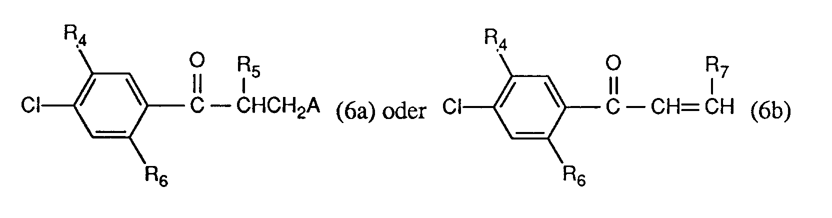
worin A Halogen, eine Di-C₂-₄-Alkylaminogruppe oder einen Morpholino-, Pyrrolidino- oder Piperidinorest bedeuten, mit Hydrazinen der Formel 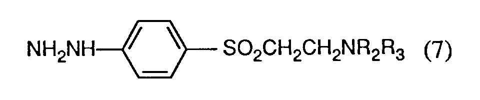
oder deren Acetonhydrazone und gegebenenfalls anschliessende Protonierung, wie oben angeführt, herstellen.

Die Protonierung erfolgt nach bekannter Art und Weise, z.B. durch Zugabe der gewünschten Säure.

Die Hydrazine der Formel (7), worin R₂ und R₃ unabhängig voneinander Methyl, Aethyl oder Hydroxyäthyl bedeuten, sowie deren Salze und Acetonhydrazone sind neu. Man erhält die Hydrazine der Formel (7) durch Behandeln des Anilins der Formel 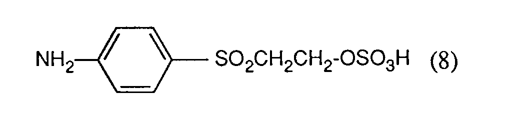
mit Natronlauge, wobei p-Vinylsulfonylanilin erhalten wird, und anschliessend Reaktion mit HNR₂R₃ zu Verbindungen der Formel 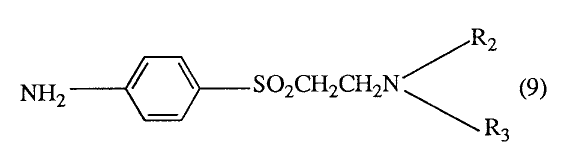
welche sodann diazotiert, reduziert und verseift werden.

Die Acetonhydrazone erhält man durch Zugabe von Aceton zu dem bei der Herstellung der Hydrazine der Formel (7) anfallenden sauren Reaktionsgemisch und anschliessende Zugabe von Alkalien bis zum leicht alkalischen Bereich.

Die erfindungsgemässen Pyrazolin-Verbindungen oder deren Mischungen eignen sich vor allem für das Aufhellen von Polyacrylnitrilfasern nach dem Ausziehverfahren. Unter Gemischen von Verbindungen der Formel (1) sind sowohl solche zu verstehen, die sich in den Substituenten R₁ bis R₆ unterscheiden bei gleichem Anion X^{⊖} als auch vorzugsweise solche Gemische, deren Verbindungen sich allein im Anion X^{⊖} unterscheiden. Sie erzeugen hohe Weisseffekte auf Polyacrylnitril sowie modifizierten Polyacrylnitril-Fasern (Courtelle Fasern). Auch auf Celluloseacetat, Cellulosetriacetat und Polyamid-Fasern sind sie sehr wirksam. Sie eignen sich besonders bei der Gelapplikation von Polyacrylnitril d.h. bei der Einarbeitung in Spinnmassen für die Herstellung im Nassspinnverfahren z.B. auf der Basis von rhodanidhaltigen Spinn- und Fällungsbädern. Die aufgehellten Polyacrylnitril-Fasern zeigen gute Echtheiten, besonders hohe Nasslichtechtheiten beim Bleichen mit Wassersuperoxid in Anwesenheit von Waschmitteln und Natriumperborat.

Die erfindungsgemässen Salze der Formel (1) bzw. (2) und insbesondere (3) können in fester Form oder in Losungsmitteln verwendet werden. Sie zeichnen sich aus durch besonders hohe Wasserlöslichkeit bei Raumtemperatur. Die Salze anderer Säuren wie z.B. die Hydrochloride, Sulfate, Phosphate, Nitrate, Oxalate, Malonate, zeigen diese Eigenschaften nicht.

Stabile Festformen lassen sich aus den nicht- oder wenig-flüchtigen Säuren wie der Phosphorigen Säure, der Sulphaminsäure, der Methansulfosäure oder der Methanphosphonsäure herstellen. Man verrührt hierzu die Pyrazolinbase mit der berechneten Menge Säure in einem geeigneten organischen Lösungsmittel bei 20-80°C, wobei sich gewöhnlich das gewünschte Salz ausscheidet und abgesaugt werden kann. Man kann die Umsetzung auch in Wasser durchführen, die erhaltene Lösung weitgehend eindampfen und das Produkt mit einem geeigneten Lösungsmittel, worin das Salz wenig löslich ist, ausfallen. Geeignete Lösungsmittel sind besonders solche, die mit Wasser mischbar sind wie Methanol, Aethanol, Isopropanol, t-Butanol, Acetonitril, Aceton, Methyläthylketon oder Dioxan.

Handelsformen kationischer Aufheller in fester Form weisen eine Anzahl negativer Eigenschaften auf, wie Stäuben, geringe Rieselfähigkeit, Knollenbildung infolge Zusammenbackens, schlechte automatische Dosierbarkeit und mangelde Lösegeschwindigkeit. Durch den Einsatz von Handelsformen in Form von Lösungen lassen sich diese Nachteile vermeiden. Solche Flüssigformulierungen sind auch besonders für den Einsatz zum optischen Aufhellen von Polyacrylfasern im Gelzustand erforderlich. Bei diesem Aufhellverfahren wird je nach Färbeaggregat und Dosieranlage mit relativ konzentrierten Aufhellerlösungen gearbeitet. Besondere Bedeutung kommt aus diesem Grunde der Löslichkeit der Aufheller und der Beständigkeit der Aufhellerlösung zu. Die Grenzen der Anforderungen an die Löslichkeit werden im wesentlichen durch die Art des Färbeaggregates bzw. der daraus resultierenden Flottenaufnahme, durch die Form der Nachdosierung sowie die Färbetemperatur. Allgemein ist festzuhalten, dass nur gut lösliche Produkte eine genügende Betriebssicherheit gewährleisten und somit für diese Applikation infrage kommen. Aufheller-Lösungen ermöglichen nämlich ein höheres Angebot und damit ein rascheres Aufziehen auf der Faser. Flüssige, lagerfähige Handelsformen möglichst hoher Konzentration sind auch aus wirtschaftlichen Gründen (Transport) erwünscht.

Ein weiterer Gegenstand der Erfindung sind wässrige Lösungen enthaltend 10-35% einer oder mehrere Pyrazolinverbindungen der Formel 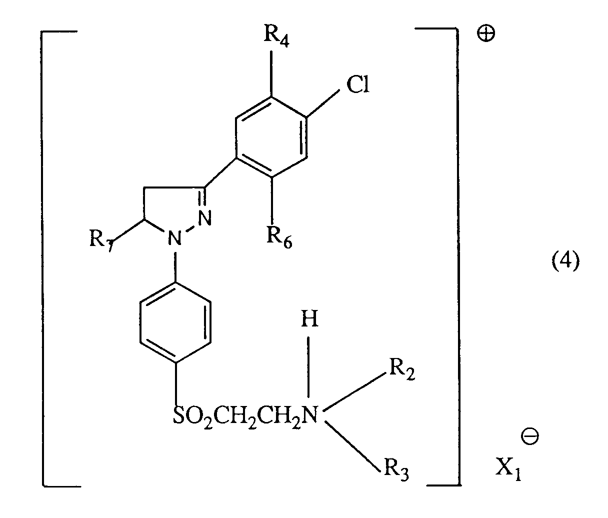
worin
R₂ und R₃ unabhängig voneinander Methyl, Ethyl oder Hydroxyethyl, vorzugsweise Methyl
R₄ Wasserstoff oder Chlor, vorzugsweise Wasserstoff
R₆ Wasserstoff oder Methyl, vorzugsweise Wasserstoff
R₇ Wasserstoff, Methyl oder Phenyl, vorzugsweise Wasserstoff und
X₁^{⊖} ein C₁-C₃-Alkanoat-, C₁-C₄Alkanphosphonat-, C₁-C₄Alkansulfonat-, Phosphit-, Sulfaminat- oder Glykolat-Anion, vorzugsweise ein C₁-C₃-Alkanoat-, C₁-C₄Alkanphosphonat-, C₁-C₄Alkansulfonat-, Phosphit- oder Sulfaminat-Anion, insbesondere ein Formiat-, Acetat-, Propionat-, Methanphosphonat-, Methansulfonat-, Phosphit- oder Sulfaminat-Anion bedeuten.

Von besonderer technischer Bedeutung sind wässrige Lösungen von einem oder mehreren Pyrazolinsalze der Formel (4), insbesondere wässrige Lösungen von einem oder mehreren der vorstehend genannten Pyrazolinsalze mit C₁-C₃-Alkancarbonsäuren, vor allem der bevorzugten Pyrazolinsalze mit Essigsäure.

Man erhält die Lösungen durch Auflösen der freien Amine in Wasser unter Beifügen der gewünschten Säure, wie Ameisensäure, Essigsäure, Propionsäure, Glykolsäure, Sulfaminsäure, Phosphorigsäure, Methanphosphonsäure, Aethanphosphonsäure, Propanphosphonsäure, Butanphosphonsäure, Methansulfonsäure, Aethansulfonsäure, Propansulfonsäure oder Butansulfonsäure. Man kann auch die als Festsubstanz isolierten Salze in Wasser lösen.

Die Salzlösungen der stärkeren Säuren, wie der Alkansulfonate, Alkanphosphonate, Formiate and vor allem der Phosphite und Sulfaminate zeichnen sich durch eine hohe Langzeit-Wärmestabilität aus, wie sie beim Lagern bei erhöhter Temperatur erforderlich ist, ohne dass dabei eine Fällung infolge Zersetzung eintritt. Zu ihrer Herstellung ist kein oder nur ein geringer Ueberschuss an Säure notwendig. Auch die festen Salzformen erleiden beim Lagern bei erhöhter Temperatur keine Zersetzung.

Die wässrigen Lösungen der leicht flüchtigen, schwachen Säuren, wie vor allem der Essigsäure, zeichnen sich durch eine gute Kältestabilität aus, wie beim Transport bei tiefer Temperatur erforderlich ist. Man verwendet sie vorzugsweise im Ueberschuss, da sie den pH-Wert nur wenig herabsetzen. Der bervorzugte pH-Bereich liegt zwischen 2 und 5. Solche Lösungen lassen sich relativ tief abkühlen ohne zu gelieren oder zu kristallisieren. Ein Zusatz von lösungsstabilisierenden oder hydrotropen Mitteln ist dabei nicht notwendig.

Eine sowohl hohe Wärme- wie Kältestabilität erreicht man insbesondere mit wässrigen Lösungen einer Pyrazolinbase mit Mischungen verschiedener Anionen. Besonders geeignet sind dabei Kombinationen einer stärkeren Säure, wie Sulfaminsäure, Phosphorige Säure, Methanphosphonsäure oder Ameisensäure mit einer schwachen Säure, insbesondere mit Essigsäure. Mit solchen Säuremischungen wird ausserdem die Wasserlöslichkeit noch einmal erhöht. So gelingt es, beim Raumtemperatur lagerstabile Lösungen ausserordentlich hoher Konzentration an Aufhellerwirksubstanz (bis 70 %) zu erzeugen, die mit den einzelnen Komponenten nicht erreichbar sind. Durch Verwendung von Säuremischungen anstelle von einzelnen Säuren erreicht man eine weitere Erniedrigung der Geliertemperatur beim Abkühlen. Es können auch Mischungen mit 3 und mehr Säurekomponenten eingesetzt werden.

Herstellungsbeispiel 1: Zu einer Mischung von 24,8 g der Verbindung der Formel 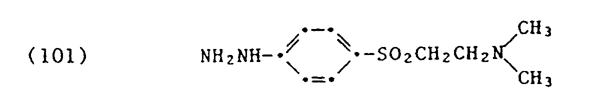
(Gehalt 98,1 %) in 30 ml Aethanol und 10 ml Wasser fügt man 19,7 g konz. Salzsäure und erwärmt auf 75°C. Alsdann tropft man bei dieser Temperatur unter Rühren im Verlauf von 1/2 Stunde eine 40°C warme Lösung von 20,9 g der Verbindung der Formel 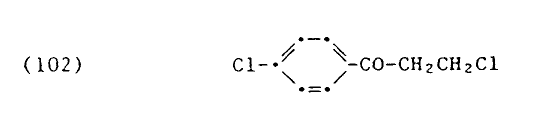
(Gehalt 97 %) in 20 ml Aethanol. Man rührt über Nacht bei Rückflusstemperatur, versetzt die, Suspension des erhaltenen Pyrazolinhydrochlorids bei 50 bis 60°C mit ca. 29 ml 30%iger wässriger Natronlauge bis zu einem konstanten pH von 11-12 und lässt abkühlen. Das ausgefallene Produkt wird abgesaugt und wiederholt mit Aethanol und dann mit Wasser gewaschen. Nach dem Trocknen im Vakuum bei 60°C erhält man 32,5 g der Verbindung der Formel 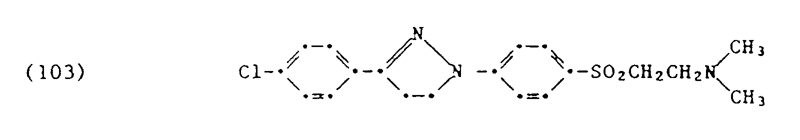
Smp. 157-158°C.

Die als Ausgangsprodukt benötigte Verbindung der Formel (101) kann man wie folgt herstellen: Zu einer Suspension von 586,6 g der Verbindung der Formel 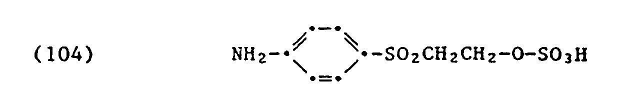
(Gehalt 95,9 %) in 2 1 Wasser tropft man unter Rühren und Kühlen bei Raumtemperatur 30%ige Natronlauge, bis ein konstanter pH von 10-11 erreicht ist (Verbrauch 420 ml). Das Ausgangsprodukt geht dabei allmählich in Lösung und danach fällt das Reaktionsprodukt aus. Man verrührt die dicke Suspension noch 1/2 Stunde bei Raumtemperatur, saugt ab und wäscht den Rückstand mit Wasser salzfrei. Man erhält 418,6 g feuchte Ware entsprechend 336,2 g getrocknetem Produkt der Verbindung der Formel 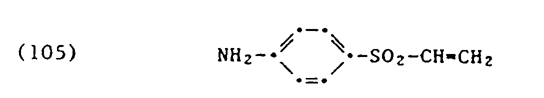
Smp. 71-72°C.

Die 418,6 g des noch feuchten Produktes werden in 500 ml Wasser verrührt. Zur erhaltenen Suspension lässt man 271 g einer wässrigen 40%igen Dimethylaminlösung zufliessen, wobei die Temperatur etwas ansteigt. Das Ausgangsprodukt geht dabei in Lösung und das Reaktionsprodukt fällt aus. Man erwärmt langsam auf 85°C, hält 1 Stunde bei 85°C, lässt abkühlen und nutscht ab. Der Rückstand wird mit 300 ml Eiswasser gewaschen und in Vakuum bei 80°C getrocknet. Man erhält 402,0 g der Verbindung der Formel 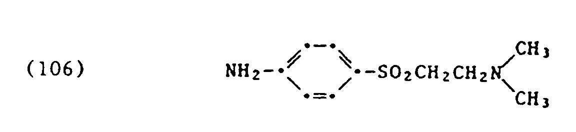
vom Smp. 135-136°C. Sie kann aus Wasser, Isopropanol oder Toluol umkristallisiert werden (Smp. 136-137°C).

Man kann die beiden vorangehend beschriebenen Reaktionsstufen auch im Eintopfverfahren durchführen ohne Zwischenisolierung der Verbindung der Formel (105).

Zu einer Suspension von 182,6 g der Verbindung der Formel (106) in 500 ml Wasser fügt man unter Rühren und Kühlen 283,5 g konz. Salzsäure. Zur erhaltenen Lösung tropft man im Verlauf von etwa ½ Stunde bei 0-5°C eine Lösung von 56 g Natriumnitrit in 100 ml Wasser. Man lässt 1 Stunde bei derselben Temperatur nachrühren, korrigiert nötigenfalls mit Sulfaminsäure bzw. Natriumnitrit auf Kaliumiodid-Stärke-Umschlag und trofpt die Diazoniumsalzlösung unter Rühren und weiterem Kühlen im Verlauf von ca. 50 Min. in 624,3 g. einer wässrigen 40%igen Natriumbisulfitlösung, die zuvor mit 30%iger Natronlauge (105 ml) auf pH 6-7 gebracht worden ist. Durch gleichzeitiges Zutropfen von weiterer Natronlauge hält man den pH bei 7. Gesamtverbrauch an Natronlauge: 183 ml. Man erwärmt die Lösung auf 60°C und tropft vorsichtig 243 g konz. Salzsäure hinzu, so dass die Schwefeldioxidgasentwicklung unter Kontrolle gehalten werden kann. Nach 4-stündigem Nachrühren bei 90°C wird die Lösung auf 0-5°C abgekühlt und mit 30%iger Natronlauge (ca. 400 ml) auf pH 10-12 gestellt. Dabei fällt das Hydrazin als freie Base aus. Es ist empfindlich gegen starke Alkalien (Violettfärbung). Man saugt ab, verrührt den Rückstand zur Reinigung in 2 l Methylenchlorid, entfernt unlösliches Salz durch Filtration, trennt die obere wässrige Phase im Scheidetrichter ab und trocknet die Methylenchloridphase. (Vorteilhaft zieht man das Hydrazin bereits aus der alkalisch gestellten wässrigen Phase zweimal mit Methylenchlorid.) Nach Abdampfen des Methylenchlorids kristallisiert man den Rückstand aus 500 ml Isopropanol, filtriert bei 5°C und wäscht zweimal mit je 50 ml eisgekühltem Isopropanol. Man erhält 160,8 g der Verbindung der Formel (101), Smp 126-128°C.

Anstelle von Methylenchlorid kann man auch Aethanol als Extraktionsmittel verwenden und die erhaltene äthanolische Lösung wie eingangs beschrieben direkt mit der Verbindung der Formel (102) umsetzen.

Anstatt das Hydrazin der Formel (101) zu isolieren, kann man die bei der Herstellung anfallende saure Reaktionslösung, die das Hydrazin (101) als Salz (Dihydrochlorid Smp. ∼ 135° (Zers.) und Dihydrosulfat) enthält, nach Alkalizugabe bis pH 2-3, auch direkt mit der Verbindung der Formel (102) umsetzen. Das zunächst ausfallende Pyrazolinsalz (hauptsächlich das Hydrochlorid der Base der Formel (103), Smp. 249-250°) wird vorteilhaft mittels Filtration isoliert und durch wiederholtes Waschen mit Aethanol gereinigt. Nach der Umsetzung mit Natronlauge in Aethanol, wie eingangs beschrieben, erhält man das Pyrazolin der Formel (103) in hoher Reinheit.

Herstellungsbeispiel 2: Eine nach Beispiel 1 aus 117,3 g der Verbindung der Formel (106) (Gehalt 97,3 %) hergestellte saure Lösung des Hydrazins der Formel (101) versetzt man bei Raumtemperatur mit 50 ml Aceton. Zu der Lösung tropft man unter Rühren 30%-ige Natronlauge (220 ml), bis ein konstanter pH-Wert von 11 erreicht ist. Das ausgefallene Produkt wird abgenutscht, mit Wasser salzfrei gewaschen und getrocknet. Man erhält 125,5 g der Verbindung der Formel 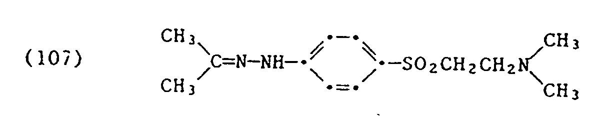
Smp. 121-122° (nach Umkristallisieren aus Isopropanol oder Toluol).

28,4 g des Acetonhydrazons der Formel (107) werden in 30 ml Wasser und 17,0 ml konzentrierter Salzsäure gelöst und die Lösung unter Rühren zum Sieden erhitzt. Im Verlauf von 2 Stunden wird ein Gemisch von 7,1 ml Aceton und Wasser abdestilliert. Man tropft alsdann bei 85°C im Verlauf ½ Stunde eine Lösung von 20,8 g der Verbindung der Formel (102)(Gehalt 98%) in 20 ml Aethanol hinzu und fährt fort gemäss Beispiel 1. Man erhält 29,3 g der Verbindung der Formel (103).

Herstellungsbeispiel 3: 10,4 g 1-(p-Vinylsulfonyl-phenyl)-3-(p-chlorhenyl)-2-pyrazolin werden in 7,2 ml 2-Methylamino-äthanol bei 130° verrührt. Nach 3 Stunden lässt man die Lösung abkühlen und verdünnt noch in der Hitze mit 50 ml Isopropanol. Man saugt den ausgefallenen Niederschlag ab, wäscht mit Isopropanol und trocknet. Man erhält 10,8 g der Verbindung der Formel 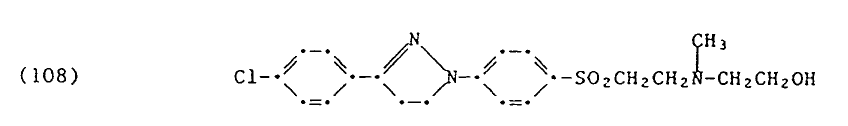
Smp. 131-132°C (nach Umkristallisation aus Perchloräthylen und Isopropanol).

In analoger Weise erhält man das 1-[p-(Dihydroxyäthyl)aminoäthyl-sulfonyl-phenyl]-3-(p-chlorphenyl)-2-pyrazolin vom Smp. 126-128°C (nach Umkristallisieren aus Toluol und Isopropanol).

Die Verbindung der Formel (108) lässt sich auch analog Beispiel 1 herstellen. Das entsprechende Hydrazin der Formel 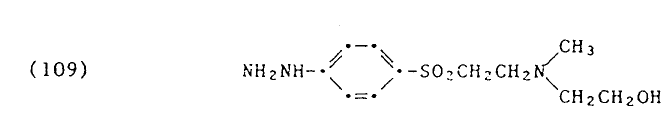
ist eine hochviskose Flüssigkeit, die beim Aufbewahren langsam erstarrt. Acetonhydrazon Smp. 114-115°C.

Herstellungsbeispiel 4: 6,9 g 1-(p-Vinylsulfonyl-phenyl)-3-(p-chlorphenyl)-2-pyrazolin werden in 5,5 g einer 33%igen äthanolischen Dimethylaminlösung und 15 ml Aethanol verrührt. Man erwärmt die Lösung allmählich im Verlauf einer Stunde auf 78°C und rührt weiter, bis sich das Ausgangsprodukt löst und eine eingedampfte Probe in verdünnter Essigsäure löslich wird. Man lässt auf Raumtemperatur abkühlen, wobei das Reaktionsprodukt dick ausfällt. Dieses wird abgesaugt, mit Aethanol gewaschen und getrocknet. Man erhält 6,7 g 1-(p-Dimethylaminoäthylsulfonyl-phenyl)-3-(p-chlorphenyl)-2-pyra-zolin (Formel 103).

In analoger Weise, aber unter Verwendung von Diäthylamin in Isopropanol anstelle von äthanolischem Dimethylamin erhält man das 1-(p-Diäthylaminoäthylsulfonyl-phenyl)-3-(p-chlorphenyl)-2-pyrazolin (Smp. 104-107°C).

Beispiel 1: 7,8 g der Verbindung der Formel (103) und 1,7 g Phosphorige Säure werden in 50 ml Aethanol 10 Minuten bei Rückflusstemperatur verrührt. Man lässt die dicke Suspension auf Raumtemperatur abkühlen, nutscht und wäscht den Rückstand wiederholt mit Aethanol. Nach dem Trocknen bei 90°C im Hochvakuum erhält man 9,1 g der Verbindung der Formel 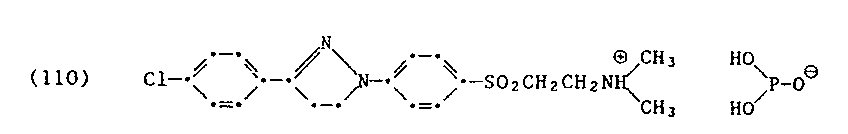
Smp. 154°C.

Beispiel 2: 7,85 g der Verbindung der Formel (103) und 1,95 g Sulfaminsäure werden in 25 ml Wasser bei Raumtemperatur verrührt. Man dampft die erhaltene Lösung bei Raumtemperatur im Hochvakuum nahezu vollständig ein und verrührt den Rückstand in ca. 50 ml Methanol. Das kristalline Produkt wird abgesaugt, wiederholt mit Methanol gewaschen und bei Raumtemperatur im Hochvakuum über Calciumchlorid getrocknet. Man erhält die Verbindung der Formel 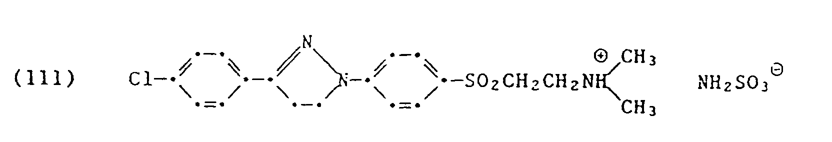
als fast farbloses Produkt (Smp. ca. 136°C, unscharf).

Beispiel 3: 7,8 g der Verbindung der Formel (103) und 2,1 g Methanphosphonsäure werden in 100 ml Isopropanol aufgekocht. Man lässt auf Raumtemperatur abkühlen, nufscht das ausgeschiedene Produkt ab, wäscht zweimal mit Isopropanol und trocknet im Hochvakuum bei Raumtemperatur. Man erhält 9,6 g der Verbindung der Formel 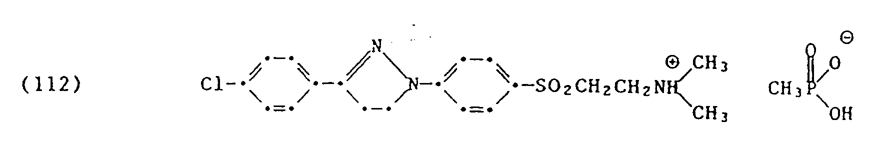
in Form nahezu farbloser wasserlöslicher Kristalle vom Smp. 146°C. Die Löslichkeit dieses Produktes in Wasser bei Raumtemperatur ist über 40 %.

Beispiel 4: 165,0 g der Base der Formel (103) und 34,5 g Phosphorige Säure werden in 500 ml Wasser bei Raumtemperatur verrührt. Zur erhaltenen Lösung fügt man noch 110 g Eisessig hinzu und verdünnt mit Wasser auf ein Gesamtgewicht von 1'100 g. Eine geringfügige Trübung durch Verunreinigung wird gegebenenfalls durch Klärfilitration entfernt. Man erhält eine 15%ige Lösung (bezogen auf das Ausgangsprodukt) vom pH 2,7 mit einer Geliertemperatur unter 0°C und einer hohen Wärmebeständigkeit beim Lagern bei 60°C.

Setzt man in diesem Beispiel anstelle der Phosphorigen Säure 40,9 g Sulfaminsäure ein so erhält man ein Produkt mit ähnlichen Eigenschaften.

Beispiel 5: Man verrührt in 4 verschiedenen Ansätzen je 50,0 g der Verbindung der Formel (103) in
a) 20,0 g Ameisensäure 85 %
   20,0 g Essigsäure
b) 12,9 g Methanphosphonsäure
   20,0 g Essigsäure
c) 12,3 g Methansulfonsäure
   20,0 g Essigsäure
d) 27,5 g Glykolsäure
   20,0 g Essigsäure
und derjenigen Menge Wasser, dass jeweils ein Gesamtgewicht von 166,6 g resultiert. Man erhält 30%-ige Lösungen (Gehalt bezüglich Ausgangsprodukt) vom pH 2,4 bis 3,0.

Beispiel 6: Gemäss Beispiel 4 erhält man aus den in den Herstellungsbeispielen 3 und 4 beschriebenen Produkten 15%ige wässrige Lösungen der Verbindungen der Formel 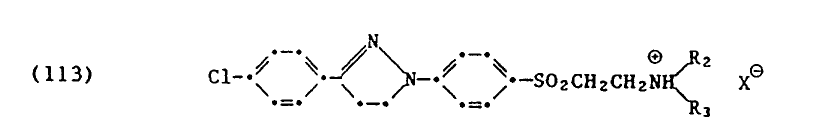
a) R₂ = CH₃, R₃ = -CH₂CH₂OH
b) R₂ = R₃ = -CH₂CH₂OH
c) R₂ = R₃ = -CH₂CH₃
worin X das Phosphit- und das Acetation bedeuten.

Beispiel 7: Ein Polyacrylintril-Gewebe (Orlon 75) wird auf einem Färbeapparat bei einem Flottenverhältnis von 1:20 mit einem wässrigen Bad, das 0,1 % des Aufhellers der Formel (110), (111), (112) oder (113 a-c),bezogen auf das Warengewicht, 1 g/l eines Anlagerungsproduktes von 35 Mol Aethylenoxid an 1 Mol Stearylalkohol und 1 ml/l Essigsäure enthält, behandelt. Die Applikation erfolgt gemäss folgendem Temperaturprogramm:
40-97°C/30 Minuten, 97°C/30 Minuten, 97-40°C/15 Minuten. Anschliessend wird das Polyacrylnitril-Gewebe während 30 Sekunden in fliessendem, enthärtetem Wasser gespült und bei 70°C im Trockenschrank getrocknet. Das so behandelte Gewebe weist einen hohen Aufhelleffekt auf.

Aehnliche Effekte erhält man bei Verwendung von modifiziertem Polyacrylnitrilgewebe (Courtelle).

Beispiel 8: Ein Gewebe aus Celluloseacetat wird im Flottenverhältnis 1:30 bis 1:40 bei 50°C in ein wässriges Bad eingebracht, das 0,15 % einer der Verbindungen der Formel (110)-(113), berechnet auf das Fasergut, enthält. Man bringt die Temperatur des Behandlungsbades auf 90 bis 95°C und hält während 20 bis 45 Minuten bei dieser Temperatur. Nach dem Spülen und Trocknen erhält man stark aufgehellte Fasern.

Beispiel 9: Polyamid, 6,6-Webtricot wird auf einem Färbeapparat bei einem Flottenverhältnis von 1:20 mit einem wässrigen Bad behandelt, welches 0,2 %, bezogen auf das Gewicht des Gewebes, einer Verbindung der Formel (110)-(113) und 1 g/l eines Alkylphenolpolyglykoläthers ethält. Man erwärmt das Bad innerhalb von 30 Minuten auf 130°C, hält es während 30 Minuten bei dieser Temperatur und kühlt dann innerhalb von 15 Minuten auf 40°C ab. Das Gewebe wird anschliessend in fliessendem deionisiertem Wasser gespült und bei 180°C mit dem Bügeleisen getrocknet. Das so behandelte Polyamidgewebe weist in allen Fällen einen hohen Aufhelleffekt auf.

Beispiel 10: Frisch ausgesponnenes Polyacrylnitril-Nasskabel (entsprechend 3,0 g Trockengewicht) wird noch feucht bei 45°C für 4 Sekunden in 100 ml einer wässrigen Flotte getaucht, die 0,0005 % eines Aufhellers der Formel (110) bis (113) enthält und mit konzentrieter Essigsäurelösung auf pH 4 eingestellt worden ist Anschliessend spült man das Nasskabel kurz mit Wasser und trocknet bei 90 bis 100°C. Man erhält auf diese Weise eine gut aufgehellte Polyacrylnitrilfaser.

Höhere Weisseffekte werden durch Erhöhen der Aufehllerkonzentration, z.B. auf 0,005 %, erzielt.

Beispiel 18: Ein im Natrium-Rhodanid-Nass-Spinnverfahren gewonnenes, gespültes und verstrecktes ungetrocknetes Polyacrylnitrilkabel wird im Flottenverhältnis 1:100 bei 45°C für 4 Sekunden in eine wässrige Lösung getaucht, die pro Liter 0,1 g eines in Beispiel 4 oder 5 beschriebenen Aufhellers und 0,5 ml Ameisensäure 85 % (pH-Wert der Lösung: 4) enthält.

Man spült anschliessend kurz in Wasser und trocknet bei 95°C an der Luft. Das so erhaltene Polyacrylnitril-Kabel weist einen starken Aufhelleffekt auf.

## Patentansprüche

1. Pyrazolinverbindungen der Formel 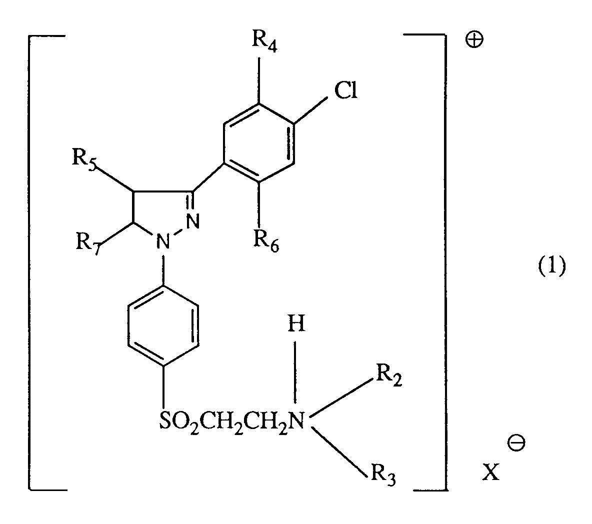 worin
R₂ und R₃ unabhängig voneinander Methyl, Ethyl oder Hydroxyethyl,
R₄ Wasserstoff oder Chlor,
R₅ und R₆ unabhängig voneinander Wasserstoff oder Methyl,
R₇ Wasserstoff, Methyl oder Phenyl, und
X^{⊖} ein Methanphosphonat-, Methansulfonat-, Phosphit- oder Sulfaminat-Anion bedeutet.

2. Pyrazolinverbindungen gemäss Anspruch 1 der Formel 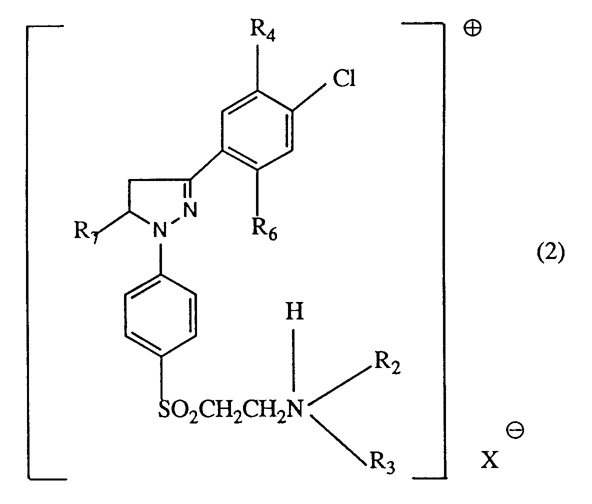 worin R₂, R₃, R₄, R₇ und X^{⊖} die in Anspruch 1 angegebene Bedeutung haben.

3. Pyrazolinverbindungen gemäss Anspruch 2 der Formel 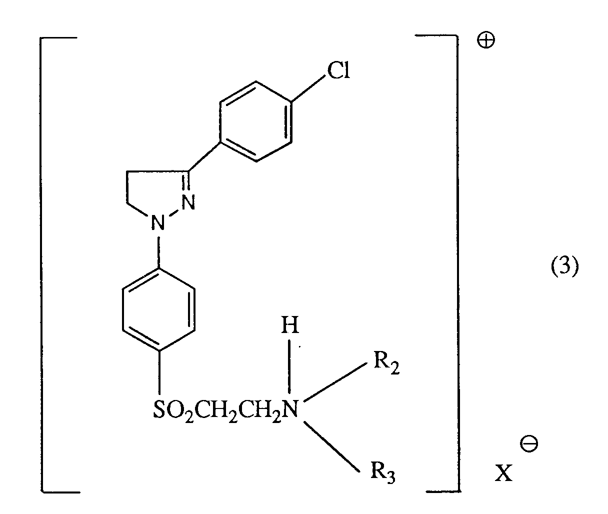 worin R₂, R₃ und X^{⊖} die in Anspruch 2 angegebene Bedeutung haben.

4. Pyrazolinverbindungen gemäss Anspruch 3, dadurch gekennzeichnet, dass R₂ und R₃ Methyl bedeuten.

5. Pyrazolinverbindungen gemäss Anspruch 4, dadurch gekennzeichnet, dass X^{⊖} ein Phosphit- oder Sulfaminat-Anion bedeutet.

6. Verwendung der Pyrazolinverbindungen gemäss Anspruch 1 oder deren Mischungen als optische Aufheller.

7. Verwendung gemäss Anspruch 6 zum optischen Aufhellen von Polyacrylnitril, Celluloseacetat oder Polyamid enthaltenden Fasern.

8. Verwendung gemäss Anspruch 7 zum optischen Aufhellen von Polyacrylnitrilfasern im Gelzustand.

9. Wässrige Lösungen enthaltend 10-35% einer oder mehrere Pyrazolinverbindungen der Formel 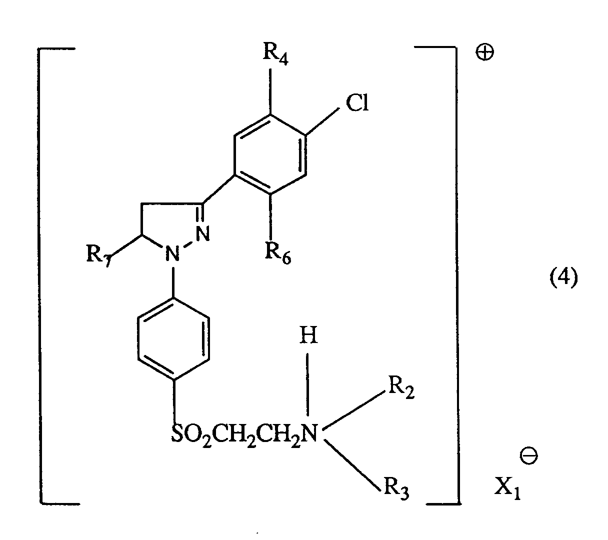 worin
R₂ und R₃ unabhängig voneinander Methyl, Ethyl oder Hydroxyethyl,
R₄ Wasserstoff oder Chlor,
R₆ Wasserstoff oder Methyl,
R₇ Wasserstoff, Methyl oder Phenyl, und
X₁^{⊖} ein C₁-C₃-Alkanoat-, C₁-C₄Alkanphosphonat-, C₁-C₄Alkansulfonat-, Phosphit-, Sulfaminat- oder Glykolat-Anion bedeuten.

10. Wässrige Lösungen gemäss Anspruch 9, dadurch gekennzeichnet, dass sie einen oder mehrere Pyrazolinverbindungen der Formel (4) enthalten, worin
R₂ und R₃ die in Anspruch 9 angegebene Bedeutung haben,
R₄, R₆ und R₇ Wasserstoff und
X^{⊖} ein C₁-C₃-Alkanoat-, C₁-C₄Alkanphosphonat-, C₁-C₄Alkansulfonat-, Phosphit-, Sulfaminat-Anion
bedeuten.

11. Wässrige Lösungen gemäss Anspruch 10, dadurch gekennzeichnet, dass sie einen oder mehrere Pyrazolinverbindungen der Formel (4) enthalten, worin
R₂ und R₃ Methyl,
R₄, R₆ und R₇ die in Anspruch 10 angegebene Bedeutung haben und
X^{⊖} ein Formiat-, Acetat-, Propionat-, Methanphosphonat-, Methansulfonat-, Phosphit- oder Sulfaminat-Anion
bedeuten.

12. Wässrige Lösungen enthaltenden 10-35% einer oder mehrere Pyrazolinverbindungen gemäss Anspruch 4 mit C₁-C₃-Alkancarbonsäuren.

13. Wässrige Lösungen enthaltenden 10-35% einer oder mehrere Pyrazolinverbindungen gemäss Anspruch 5 mit Essigsäure.

## Claims

1. Pyrazoline compounds of formula 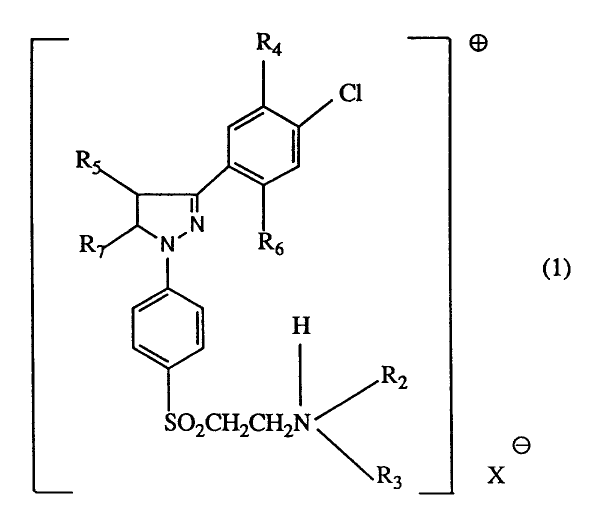 wherein
R₂ and R₃ are each independently of the other methyl, ethyl or hydroxyethyl,
R₄ is hydrogen or chlorine,
R₅ and R₆ are each independently of the other hydrogen or methyl,
R₇ is hydrogen, methyl or phenyl, and
X^{⊖} is a methanephosphonate-, methanesulfonate-, phosphite- or sulfaminate anion.

2. Pyrazoline compounds according to claim 1 of formula 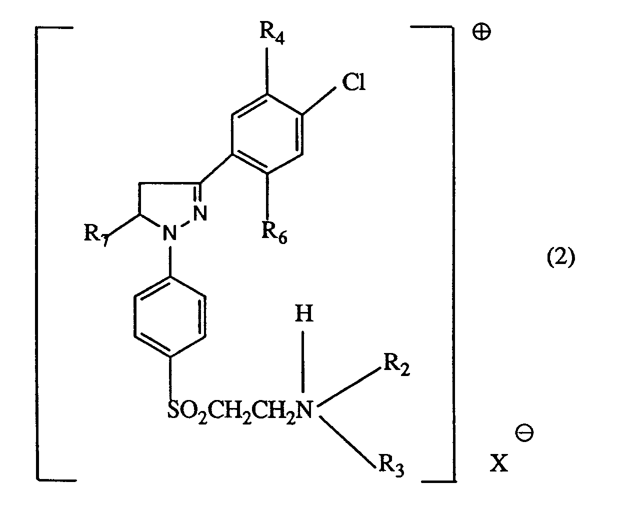 wherein R₂, R₃, R₄, R₇ and X^{⊖} are as defined in claim 1.

3. Pyrazoline compounds according to claim 2 of formula 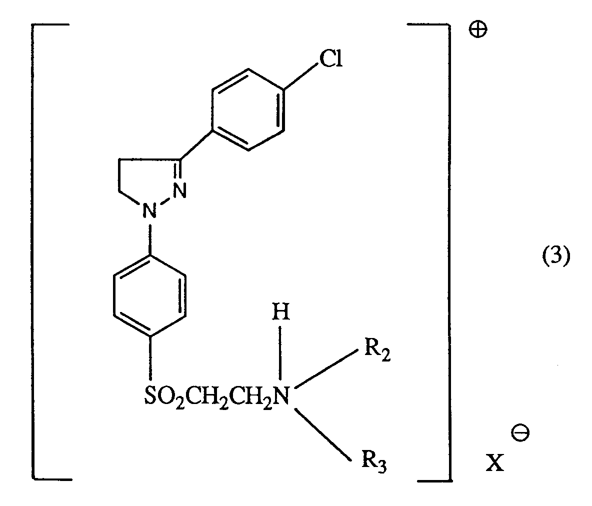 wherein R₂, R₃ and X^{⊖} are as defined in claim 2.

4. Pyrazoline compounds according to claim 3 wherein both R₂ and R₃ are methyl.

5. Pyrazoline compounds according to claim 4 wherein X^{⊖} is a phosphite- or sulfaminate anion.

6. Use of the pyrazoline compounds according to claim 1 or of a mixture thereof as fluorescent whitening agents.

7. Use according to claim 6 for whitening fibres comprising polyacrylonitrile, cellulose acetate or polyamide.

8. Use according to claim 7 for whitening polyacrylonitrile fibres in the gel state.

9. Aqueous solutions containing 10-35% of one or more pyrazoline compounds of formula 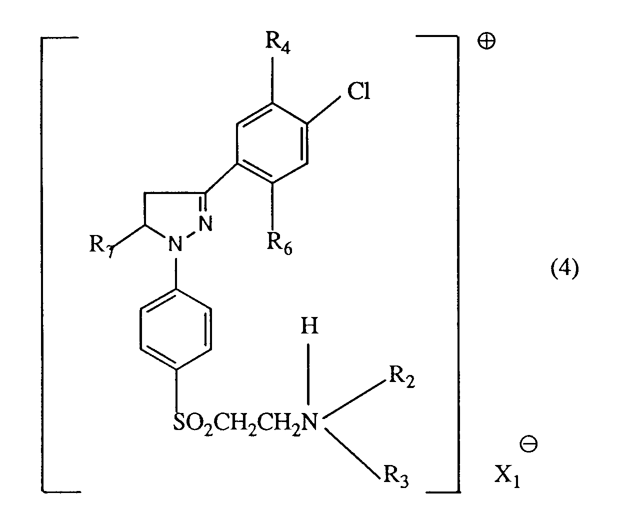 wherein
R₂ and R₃ are each independently of the other methyl, ethyl or hydroxyethyl,
R₄ is hydrogen or chlorine,
R₆ is hydrogen or methyl,
R₇ is hydrogen, methyl or phenyl, and
X₁^{⊖} is a C₁-C₃-alkanoate-, C₁-C₄-alkanephosphonate-, C₁-C₄-alkanesulfonate-, phosphite-, sulfaminate- or glykolate anion.

10. Aqueous solutions according to claim 9 containing one or more pyrazoline compounds of formula (4), wherein
R₂ and R₃ are as defined in claim 9,
R₄, R₆ and R₇ are hydrogen and
X₁^{⊖} is a C₁-C₃-alkanoate-, C₁-C₄-alkanephosphonate-, C₁-C₄-alkanesulfonate-, phosphite- or sulfaminate anion.

11. Aqueous solutions according to claim 10 containing one or more pyrazoline compounds of formula (4), wherein
R₂ and R₃ are methyl,
R₄, R₆ und R₇ are as defined in claim 10 and
X₁^{⊖} is a formate-, acetate-, propionate-, methanephosphonate-, methanesulfonate-, phosphite- or sulfaminate anion.

12. Aqueous solutions containing 10-35% of one or more pyrazoline compounds according to claim 4 with C₁-C₃-alkanecarboxylic acids.

13. Aqueous solutions containing 10-35% of one or more pyrazoline compounds according to claim 5 with acetic acid.

## Revendications

1. Composés pyrazoline de formule 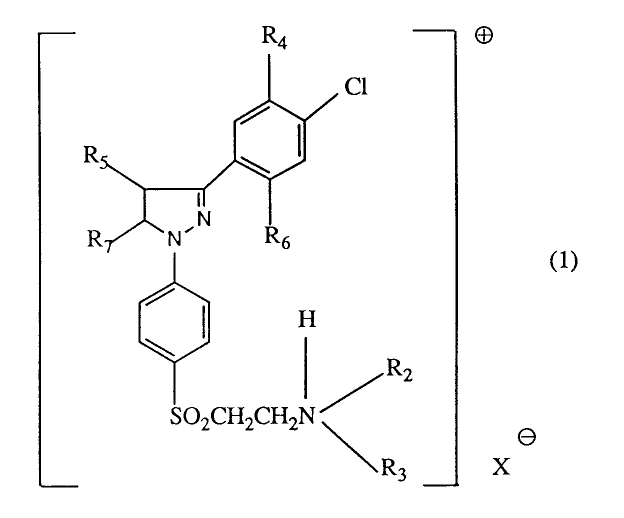 dans laquelle
R₂ et R₃ représentent chacun, indépendamment l'un de l'autre, un groupe méthyle, éthyle ou hydroxyéthyle,
R₄ représente un atome d'hydrogène ou de chlore,
R₅ et R₆ représentent chacun, indépendamment l'un de l'autre, un atome d'hydrogène ou un groupe méthyle,
R₇ représente un atome d'hydrogène ou un groupe méthyle ou un groupe phényle, et
X^{⊖} représente un anion méthanephosphonate, méthanesulfonate, phosphite ou sulfaminate.

2. Composés pyrazoline conformes à la revendication 1, de formule 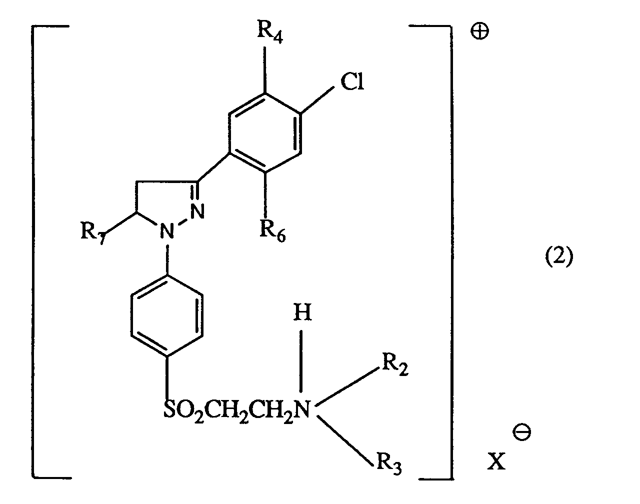 dans laquelle R₂, R₃, R₄, R₇ and X^{⊖} ont les significations indiquées dans la revendication 1.

3. Composés pyrazoline conformes à la revendication 2, de formule 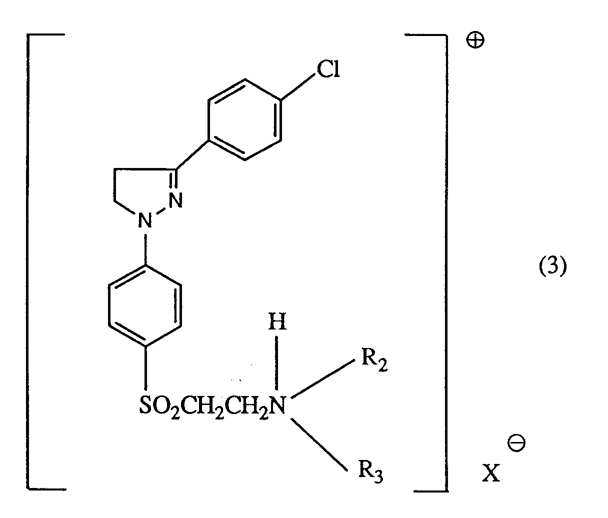 dans laquelle R₂, R₃ und X^{⊖} ont les signinifications indiquées dans la revendication 2.

4. Composés pyrazoline conformes à la revendication 3 dans laquelle R₂ et R₃ représentent un groupe méthyle.

5. Composés pyrazoline conformes à la revendication 4 dans laquelle X^{⊖} représente un anion phosphite ou sulfaminate.

6. Utilisation des composés pyrazoline conformes à la revendication 1, ou de leurs mélanges, comme azurant optique.

7. Utilisation selon la revendication 6, pour l'azurage optique de fibres contenant un polyacrylonitrile, un acétate de cellulose ou un polyamide.

8. Utilisation selon la revendication 7, pour l'azurage optique de fibres de polyacrylonitrile, à l'état de gel.

9. Solutions aqueuses contenant 10-35% d'un ou plusieurs composés pyrazoline de formule 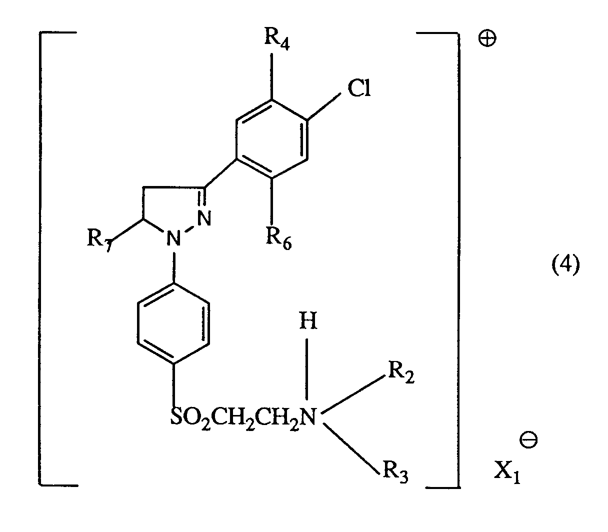 dans laquelle
R₂ et R₃ représentent chacun, indépendamment l'un de l'autre, un groupe méthyle, éthyle ou hydroxyéthyle,
R₄ représente un atome d'hydrogène ou de chlore,
R₆ représente un atome d'hydrogène ou un groupe méthyle,
R₇ représente un atome d'hydrogène ou un groupe méthyle ou un groupe phényle, et
X₁^{⊖} représente un anion C₁-C₃-alcanoate, C₁-C₄-alcanephosphonate, C₁-C₄-alcanesulfonate, phosphite, sulfaminate ou glycolate.

10. Solutions aqueuses selon la revendication 9 contenant d'un ou plusieurs composés pyrazoline de formule (4), dans laquelle R₂ and R₃ ont les significations indiquées dans la revendication 9,
R₄, R₆ et R₇ représentent un atome d'hydrogène et
X₁^{⊖} représente un anion C₁-C₃-alcanoate, C₁-C₄-alcanephosphonate, C₁-C₄-alcanesulfonate, phosphite ou sulfaminate.

11. Solutions aqueuses conformes à la revendication 10 contenant d'un ou plusieurs composés pyrazoline de formule (4), dans laquelle
R₂ et R₃ représentent un groupe méthyle,
R₄, R₆ et R₇ ont les significations indiquées dans la revendication 10 et
X₁^{⊖} représente un anion formiate, acétate, propionate, methanephosphonate, methanesulfonate, phosphite ou sulfaminate,

12. Solutions aqueuses contenant 10-35% d'un ou plusieurs composés pyrazoline conformes à la revendication 4, avec des acides alcanecarboxyliques en C₁-₃.

13. Solutions aqueuses contenant 10-35% d'un ou plusieurs composés pyrazoline conformes à la revendication 5 avec de l'acide acétique.
